# EUROPÄISCHE PATENTSCHRIFT

(11) **EP 1 230 144 B1**
(45) Veröffentlichungstag und Bekanntmachung des Hinweises auf die Patenterteilung: **17.03.2004**
(21) Anmeldenummer: 00974364.2
(22) Anmeldetag: 04.09.2000
(51) Int. Cl.: B67C 7/00

(54) **VORRICHTUNG UND VERFAHREN ZUM STERILEN BEFÜLLEN VON BEHÄLTERN**
DEVICE AND METHOD FOR FILLING CONTAINERS IN A STERILE MANNER
DISPOSITIF ET PROCEDE DE REMPLISSAGE STERILE DE RECIPIENTS

(30) Priorität: 07.09.1999 DE 29923540 U; 23.08.2000 DE 10041319
(43) Veröffentlichungstag der Anmeldung: 14.08.2002
(73) Patentinhaber: Alcoa Deutschland GmbH Verpackungswerke, 67547 Worms (DE)
(72) Erfinder: SPATZ, Günther, 68623 Lampertheim (DE); SCHWARZ, Wolfhard, 67549 Worms (DE); SPETHER, Karl-Heinz, 68549 ILVESHEIM (DE)
(74) Vertreter: Gleiss, Alf-Olav, Dipl.-Ing.
(86) Internationale Anmeldenummer: PCT/EP2000/008614
(87) Internationale Veröffentlichungsnummer: WO 2001/017891

(56) Entgegenhaltungen:
- EP-A- 0 758 624
- EP-A- 0 893 397
- WO-A-98/30491
- WO-A-99/08933
- DE-A- 4 018 142
- DE-A- 4 407 183
- DE-A- 19 806 520

## Beschreibung

Die Erfindung betrifft eine Vorrichtung zum Abfüllen von Behältern, insbesondere von Flaschen, sowie ein Verfahren zum Abfüllen von Behältern, insbesondere von Flaschen.

Vorrichtungen und Verfahren der hier angesprochenen Art sind bekannt WO 98 30491 A beschreibt eine Vorschließstation und eine zweite Verschließstation außerhalb des Reinraums. Es gibt aber keinen Hinweis auf die Verwendung mehr teiliger verchlusse. Es hat sich jedoch gezeigt, dass insbesondere beim Abfüllen von verderblichem Gut, beispielsweise beim Abfüllen von Bier, Fruchtsäften oder von Mineralwasser ohne Kohlensäure, das abgefüllte Gut verunreinigt wird und damit schnell verdirbt, und somit nicht mehr für den Verzehr geeignet ist.

Es ist daher Aufgabe der Erfindung, eine Vorrichtung und ein Verfahren zu schaffen, die diese Nachteile nicht aufweisen.

Zur Lösung dieser Aufgabe wird eine Vorrichtung vorgeschlagen, die die in Anspruch 5 genannten Merkmale aufweist. Sie zeichnet sich durch einen Reinraum aus, in dem die Behälter befüllt werden. Mit Reinraum ist hier eine Umgebung gemeint, innerhalb derer die Anzahl von Keimen sehr gering ist und die praktisch frei von für das verderbliche Gut schädlichen Substanzen ist. In dem Reinraum ist auch eine Verschließstation vorgesehen. Die Behälter werden somit in dem Reinraum mit einem Verschluss verschlossen, so dass bei der Überführung von der Abfüllstation zur Verschließstation keine Keime in den Behälter gelangen können. Dem Reinraum ist zumindest eine erste Reinigungsschleuse zugeordnet, in der die Behälter vor Eintritt in den Reinraum gereinigt werden.

Bevorzugt wird ein Ausführungsbeispiel der Vorrichtung, das sich dadurch auszeichnet, dass eine zweite Reinigungsschleuse vorgesehen ist, die der Reinigung der Verschlüsse dient, die auf die Behälter aufgebracht werden. Damit ist auch hier sichergestellt, dass keine Keime oder dergleichen in den Reinraum eingeschleppt werden.

Weitere Ausgestaltungen ergeben sich aus den übrigen abhängigen Ansprüchen.

Zur Lösung der Aufgabe wird auch ein Verfahren vorgeschlagen, welches die in Anspruch 1 genannten Merkmale aufweist. Es zeichnet sich dadurch aus, dass die Behälter über eine erste Reinigungsschleuse, in der sie gereinigt werden, einem Reinraum zugeführt werden, innerhalb dessen sie befüllt werden. Die Behälter werden im Reinraum auch verschlossen, um das Einschleppen von Keimen oder sonstigen Substanzen in das verderbliche Gut zu vermeiden.

Weitere Ausführungsformen des Verfahrens ergeben sich aus den übrigen abhängigen Ansprüchen.

Ausführungsbeispiele der Erfindung werden im folgenden anhand der Zeichnung näher erläutert. Die einzige Figur zeigt eine Prinzipskizze in Form eines Blockschaltbilds der Vorrichtung zum Abfüllen von Behältern.

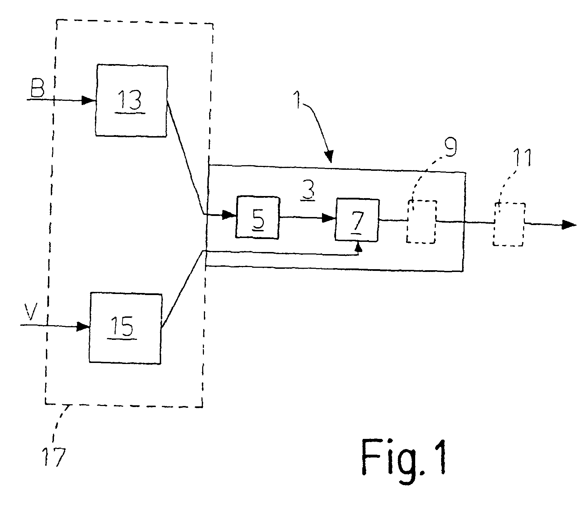

In Figur 1 ist eine Vorrichtung 1 angedeutet, innerhalb derer Behälter B mit einem verderblichen Gut befüllt werden. Die Vorrichtung 1 weist einen Reinraum 3 auf, der sich dadurch auszeichnet, dass die Anzahl der Keime pro Kubikmeter Luft und sonstige das verderbliche Gut beeinträchtigende Substanzen auf ein Minimum reduziert sind.

Innerhalb der Vorrichtung 1 ist eine Abfüllstation 5 vorgesehen, in der das verderbliche Gut in die Behälter B eingebracht wird. Die hier dargestellte Vorrichtung 1 umfasst außerdem eine Verschließstation 7, innerhalb derer Verschlüsse V auf die gefüllten Behälter B aufgebracht werden. Die Verschließstation 7 ist vorzugsweise ebenfalls im Reinraum 3, also innerhalb der Vorrichtung 1 angeordnet, um zu vermeiden, dass bei der Überführung der Behälter B von der Abfüllstation 5 zur Verschließstation 7 Keime oder sonstige Substanzen das verderbliche Gut gefährden.

Gestrichelt ist in Figur 1 innerhalb der Vorrichtung 1 eine Nachreinigungsstation 9 eingezeichnet. Sie dient dazu, die befüllten Behälter B zu reinigen, so dass an deren Außenseite kein verderbliches Gut oder sonstige Substanzen anhaften. In Figur 1 ist angedeutet, dass die Nachreinigungsstation auch außerhalb der Vorrichtung 1 vorgesehen sein kann. Die außerhalb der Vorrichtung 1 angeordnete Reinigungsstation ist mit der Bezugsziffer 11 gekennzeichnet.

Aus Figur 1 ist ersichtlich, dass eine erste Reinigungsschleuse 13 vorgesehen ist, der die zu befüllenden Behälter B zugeführt werden. Innerhalb der Reinigungsschleuse 13 werden die Behälter B gereinigt, vorzugsweise keimfrei gemacht. Die Schleuse kann eine Strahleinrichtung und/oder eine Begasungseinrichtung umfassen, wobei die Strahleinrichtung eine Flüssigkeit auf die zu reinigenden Behälter B aufsprühen und/oder eine UV- beziehungsweise radioaktive Strahlung abgeben kann. Derartige Einrichtungen sind bekannt, so dass auf deren Beschreibung hier verzichtet wird.

Die gereinigten Behälter B werden von der ersten Reinigungsschleuse 13 so der Vorrichtung 1 zugeführt, dass eine Verunreinigung der Behälter B ausgeschlossen ist. Die erste Reinigungsschleuse 13 kann daher auch unmittelbar an die Vorrichtung 1 angedockt sein.

Figur 1 zeigt außerdem eine zweite Reinigungsschleuse 15, der die Verschlüsse V zugeführt werden, mit denen die Behälter B zu verschließen sind. Innerhalb der zweiten Reinigungsschleuse 15 werden die Verschlüsse V gereinigt beziehungsweise desinfiziert, wobei auch hier eine Strahleinrichtung der obengenannten Art und/oder eine Begasungseinrichtung eingesetzt werden kann.

Von der zweiten Reinigungsschleuse 15 werden die Verschlüsse V der Vorrichtung 1 so zugeführt, dass eine Verunreinigung durch Keime oder sonstige Substanzen ausgeschlossen ist.

Durch eine gestrichelte Linie 17 ist angedeutet, dass einerseits die erste und zweite Reinigungsschleuse 13 und 15 zu einer einzigen Schleuse zusammenfasst werden können, andererseits, dass beide Schleusen unmittelbar mit der Vorrichtung 1 verbunden sein können, um eine sichere, das heißt gegen Verunreinigungen geschützte Überführung der gereinigten Gegenstände gewährleisten zu können.

Die zu reinigenden Gegenstände, die Behälter B. und die Verschlüsse V, können also in den zugehörigen Reinigungsschleusen durch eine Begasungseinrichtung, die beispielsweise Ozon abgibt, oder mittels einer Strahleinrichtung gereinigt werden. Dabei kann die Strahleinrichtung eine Reinigungsflüssigkeit auf die zu reinigenden Gegenstände abgeben, oder aber eine UV-Strahlung beziehungsweise eine radioaktive Strahlung. Es ist möglich, anschließend an die Reinigung mittels einer Spülflüssigkeit eine Nachreinigung mit einem neutralen Medium vorzunehmen, um alle Reste der Reinigungsflüssigkeit zu beseitigen. Die Nachreinigung erfolgt vorzugsweise außerhalb des Reinraumes, der dadurch relativ kompakt ausgebildet sein kann.

Im Folgenden wird auf die Funktion der Vorrichtung 1 und auf das Verfahren zum Abfüllen von Behältern näher eingegangen:

Im Inneren der Vorrichtung 1, nämlich im Reinraum 3 werden Behälter B in einer Abfüllstation 5 mit verderblichem Gut gefüllt, beispielsweise mit Fruchtsäften oder mit Mineralwasser, das einen geringen Kohlensäuregehalt hat oder frei von Kohlensäure ist. Es fehlt in diesem Fall also an der desinfizierenden Wirkung der Kohlensäure.

Um die Einbringung von Keimen in die Behälter B zu vermeiden, werden die Behälter B und vorzugsweise auch die Verschlüsse V in Reinigungsschleusen 13 und 15 beziehungsweise gegebenenfalls auch in einer gemeinsamen Reinigungsschleuse (siehe die Linie 17) gereinigt beziehungsweise desinfiziert. Die Behälter B werden anschließend der Abfüllstation 5 zugeführt, die Verschlüsse V der Verschließstation 7. Es ist auf diese Weise möglich, verderbliches Gut in die Behälter B einzufüllen und das Einschleppen von Keimen oder sonstigen störenden Substanzen zu vermeiden. Mit Hilfe der Vorrichtung 1 und des hier erläuterten Verfahrens können also Behälter B abgefüllt werden, ohne dass eine Erhitzung des einzubringenden Gutes erforderlich wäre. Einerseits trägt dies dazu bei, dass Geschmack und Inhaltsstoffe, insbesondere Vitamine, des abzufüllenden Guts nicht nachteilig beeinflusst werden, andererseits kann die Energie zum Erhitzen des Guts eingespart werden. Vorrichtung und Verfahren tragen letztlich dazu bei, dass das Mindesthalcbarkeitsdatum sicher erreicht und in vielen Fällen auch -gegebenenfalls erheblich- verlängert werden kann.

Die verschlossenen Behälter B können noch innerhalb des Reinraums 3 in einer Nachreinigungsstation 9 gereinigt werden. Es ist aber zweckmäßig, die Nachreinigung außerhalb der Vorrichtung 1 beziehungsweise des Reinraums 3 in einer Nachreinigungsstation 11 durchzuführen.

Insgesamt wird deutlich, dass die Vorrichtung 1 sehr einfach aufgebaut ist und dass auf übliche Reinigungs- und Desinfizierverfahren zurückgegriffen werden kann, um die in den Reinraum 3 eingebrachten Gegenstände, die Behälter B und gegebenenfalls auch die Verschlüsse V so zu reinigen und zu desinfizieren, dass keine Keime oder sonstigen Substanzen in das verderbliche Gut eingeschleppt werden.

Aus den Erläuterungen wird auch deutlich, dass auf einfache Weise Reinigungsschleusen 13, 15 realisiert werden können, die unmittelbar in die Gehäusewandung der Vorrichtung 1 integrierbar beziehungsweise an diese anbaubar sind. Keime können nicht in den Reinraum 3 beziehungsweise in den Bereich jenseits der Gehäusewandung gelangen, da der einzige Zugang durch die Reinigungsschleusen verläuft.

Nach allem wird deutlich ersichtlich, dass bei der hier beschriebenen Vorrichtung beziehungsweise bei Durchführung des erläuterten Verfahrens in einem Reinraum Behälter befüllt und verschlossen werden. Dabei wird mit Hilfe von mindestens einer Reinigungsschleuse sichergestellt, dass die in den Reinraum eingebrachten Gegenstände, hier also die Behälter und die Verschlüsse gereinigt sind, so dass Keime nicht eingetragen werden können. Es ist ohne weiteres ersichtlich, dass eine Reinigungsschleuse verwendet werden kann, die sowohl für die Behälter als auch für die Verschlüsse vorgesehen ist, dass aber auch für beide Elemente getrennte Reinigungsschleusen vorgesehen werden können.

Die Vorrichtung und das Verfahren sind für Behälter und Verschlüsse aller Art geeignet. Es hat sich jedoch herausgestellt, dass in einigen Fällen nicht ohne weiteres erreichbare Bereiche bei Verschlüssen vorhanden sind, in denen möglicherweise noch Keime existieren. Es werden daher besondere Verschlussarten eingesetzt, die sehr einfach aufgebaut sind, also bei einem Reinigungsvorgang sehr leicht vollständig von Keimen befreit werden können.

Bei den besonderen Verschlüssen der hier angesprochenen Art handelt es sich quasi um einfache, auch als Dichtungskappen bezeichnete Verschlusskappen, die im Inneren des Reinraums auf den Behälter aufgesetzt werden können und diesen sicher verschließen. Die Behälter werden dann außerhalb des Reinraums endgültig verschlossen, indem auf die Verschlusskappe ein Verschlusselement aufgebracht wird. Dabei kann es sich um einen herkömmlichen Kunststoff-Schraubverschluss, um einen Kronkorken, einen Kron-Drehkorken oder aber um einen üblichen Metall-Schraubverschluss handeln. Schraubverschlüsse dieser Art werden auf den mit der Verschlusskappe versehenen Behälter aufgesetzt und dann einem Formungsvorgang unterworfen, bei dem in den Mantel des Schraubverschlusses ein Gewinde eingerollt wird. Derartige Formverfahren sind allgemein bekannt, so dass hier nicht näher darauf eingegangen wird.

Wesentlich ist eben, dass vorzugsweise einfach aufgebaute Verschlusskappen eingesetzt werden, die keine Hinterschneidungen beziehungsweise Bereiche aufweisen, in denen sich auch bei einem Reinigungsvorgang Keime oder dergleichen halten können, die das Füllgut gefährden. Die Behälter lassen sich mit den Verschlusskappen sicher verschließen, so dass auch nach dem Ausfahren aus dem Reinraum eine Verunreinigung des Behälterinhalts sicher vermieden wird. Die Behälter sind dann einfach und ohne Gefährdung des Inhalts handhabbar und können auf die oben genannte Art endgültig verschlossen werden. Die Verschlusskappen können aus Kunststoff, aus mit Kunststoff beschichtetem Metall oder aus mehreren Materialien bestehen.

Vorzugsweise werden die Verschlusskappen in einer definierten Orientierung der Reinigungsschleuse so zugeführt, dass sich bei der Reinigung mit einem flüssigen Reinigungs- beziehungsweise Desinfektionsmittel keine Flüssigkeitsreste in der Verschlusskappe sammeln können.

Der einfache Aufbau der Verschlusskappen, die eben keine Hinterschneidungen oder dergleichen aufweisen, ist auch für Reinigungsverfahren mit Strahlen aller Art vorteilhaft, weil alle Bereiche der Verschlusskappe sicher erreicht werden können und Keime und dergleichen abgetötet werden.

Bei der Reinigung der unter einer besonders gewählten Orientierung gehaltenen Verschlusskappen kann sichergestellt werden, dass Reinigungsflüssigkeit sich nicht in Vertiefungen oder dergleichen ansammelt. Dadurch können an den Reinigungsvorgang anschließende Trockenverfahren besonders effektiv und zeitsparend durchgeführt werden.

Bei der Reinigung der Behälter und der Verschlüsse beziehungsweise Verschlusskappen ist sicherzustellen, dass die Materialien der zu reinigenden Teile nicht beeinträchtigt werden und dass auch der Geschmack der in den Behälter eingefüllten Substanzen beziehungsweise Flüssigkeiten nicht nachteilig beeinflusst wird. Zur Reinigung können beispielsweise Peressig und/oder Alkohol verwendet werden.

Die hier beschriebene Vorrichtung und das erläuterte Verfahren können besonders effektiv zum Abfüllen von leicht verderblichem Gut eingesetzt werden, insbesondere von Bier, Fruchtsäften und von Mineralwasser mit einem geringen Kohlensäureanteil oder ohne Kohlensäure. Dabei werden die beschriebenen als Dichtkappen bezeichneten Verschlusskappen eingesetzt, weil diese sehr gründlich gereinigt werden können.

## Patentansprüche

1. Verfahren zum Abfüllen von Behältern, insbesondere von Flaschen, mit verderblichem Gut, mit folgenden Schritten: Einbringen der Behälter in eine erste Reinigungsschleuse (13), Überführen der Behälter aus der ersten Reinigungsschleuse in einen Reinraum (3), Befüllen der Behälter im Reinraum (3), Vorverschließen der Behälter im Reinraum mit einem ersten Teil eines Verschlusses, nämlich mit einer Dichtungskappe, und endgültiges Verschließen der Behälter außerhalb des Reinraums (3) mittels eines zweiten Teils des Verschlusses.

2. Verfahren nach Anspruch 1, **dadurch gekennzeichnet, dass** die Verschlüsse einer zweiten Reinigungsschleuse zugeführt und dann an den Reinraum weitergeleitet werden, wo sie auf die Behälter aufgesetzt werden.

3. Verfahren nach Anspruch 1 oder 2, **dadurch gekennzeichnet, dass** die Behälter und/oder die Verschlüsse in der/den Reinigungsschleuse(n) mittels einer Strahleinrichtung und/oder mittels einer Begasungseinrichtung gereinigt werden.

4. Verfahren nach einem der vorhergehenden Ansprüche 1 bis 3, **dadurch gekennzeichnet, dass** die zu reinigenden Gegenstände mittels Flüssigkeitsstrahlen, UV-Strahlen, mittels radioaktiver Strahlen und/oder mittels eines Gases -insbesondere Ozongereinigt werden.

5. Vorrichtung zum Durchführen des Verfahrens nach einem der Ansprüche 1 bis 4, mit einem Reinraum (3) in dem die Behälter (B) befüllt und in einer Vorverschließstation (7) mit einem ersten Teil eines Verschlusses, nämlich mit einer Dichtungskappe, vorverschlossen werden können, und durch eine erste Reinigungsschleuse (13) für die Behälter (B), die dem Reinraum (3) so zugeordnet ist, dass die Behälter (B) vor Eintritt in den Reinraum (3) gereinigt werden und einer Verschließstation außerhalb des Reinraums (3) mit der die Behälter (B) dann endgültig mit einem zweiten Teil des Verschlusses verschlossen werden können.

6. Vorrichtung nach Anspruch 5, **dadurch gekennzeichnet, dass** eine zweite Reinigungsschleuse (15) für die Verschlüsse (V) vorgesehen ist, die dem Reinraum (3) so zugeordnet ist, dass die Verschlüsse (V) vor Eintritt in den Reinraum (3) gereinigt werden.

7. Vorrichtung nach Anspruch 5 oder 6, **dadurch gekennzeichnet, dass** die erste und/oder die zweite Reinigungsschleuse (13;15) eine Strahleinrichtung und/oder eine Begasungseinrichtung umfasst.

8. Vorrichtung nach einem der Ansprüche 5 bis 7, **dadurch gekennzeichnet, dass** die Strahleinrichtung eine Flüssigkeit, eine UV-, eine radioaktive Strahlung und/oder Gas -insbesondere Ozon- auf die zu reinigenden Gegenstände abstrahlt.

9. Vorrichtung nach einem der Ansprüche 5 bis 8, **dadurch gekennzeichnet, dass** eine Nachreinigungsstation (9;11) vorgesehen ist.

## Claims

1. Method for filling containers, in particular bottles, with perishable goods, consisting of the following steps: placing of the containers in a first cleaning sluice (13), transferring the containers from the first cleaning sluice into a clean room (3), filling the containers in the clean room (3), pre-closing the containers in the clean room with a first part of a seal, namely with a sealing cap, and final closing of the containers outside the clean room (3) by means of a second part of the seal.

2. Method according to claim 1, **characterised in that** the seals are conducted into a second cleaning sluice and then passed on to the clean room, where they are placed on the containers.

3. Method according to claim 1 or 2, **characterised in that** the containers and/or the seals are cleaned in the cleaning sluice(s) by means of a radiation device and/or by means of a gassing device.

4. Method according to one of the preceding claims 1 to 3, **characterised in that** the objects to be cleaned are cleaned by means of liquid jets, UV rays, by means of radioactive rays and/or by means of a gas, in particular ozone.

5. Device to realise the method according to one of claims 1 to 4, with a clean room (3) in which the containers (B) can be filled and pre-sealed in a pre-sealing station (7) with a first part of a seal, namely with a sealing cap, and by a first cleaning sluice (13) for the containers (B) which is arranged with respect to the clean room (3) such that the containers (B) are cleaned before entering the clean room (3) and a sealing station outside the clean room (3) with which the containers (B) can then be finally sealed with a second part of the seal.

6. Device according to claim 5, **characterised in that** a second cleaning sluice (15) is provided for the seals (V) which is arranged with respect to the clean room (3) such that the seals (V) are cleaned before entering the clean room (3).

7. Device according to claim 5 or 6, **characterised in that** the first and/or the second cleaning sluice (13; 15) includes a radiation device and/or a gassing device.

8. Device according to one of claims 5 to 7, **characterised in that** the radiation device emits a liquid, a UV radiation, a radioactive radiation and/or gas - in particular ozone - onto the objects to be cleaned.

9. Device according to one of claims 5 to 8, **characterised in that** an after-purification station (9;11) is provided.

## Revendications

1. Procédé de remplissage de récipients, en particulier de bouteilles, avec des matières périssables, comprenant les étapes suivantes : admission des récipients dans un premier sas de nettoyage (13), transfert des récipients hors du premier sas de nettoyage dans une salle blanche (3), remplissage des récipients dans la salle blanche (3), pré-fermeture des récipients dans la salle blanche avec une première partie d'un système de fermeture, à savoir un opercule d'étanchéité, et fermeture définitive des récipients à l'extérieur de la salle blanche (3) au moyen d'une deuxième partie du système de fermeture.

2. Procédé selon la revendication 1, **caractérisé en ce que** les systèmes de fermeture sont amenés dans un deuxième sas de nettoyage et sont ensuite acheminés dans la salle blanche, dans laquelle ils sont posés sur les récipients.

3. Procédé selon la revendication 1 ou 2, **caractérisé en ce que** les récipients et/ou les systèmes de fermeture sont nettoyés dans le/les sas de nettoyage au moyen d'un dispositif de projection et/ou au moyen d'un dispositif d'injection de gaz.

4. Procédé selon l'une quelconque des revendications précédentes 1 à 3, **caractérisé en ce que** les objets à nettoyer sont nettoyés au moyen de jets de liquide, de rayons UV, au moyen de rayons radioactifs et/ou au moyen d'un gaz, en particulier l'ozone.

5. Dispositif destiné à la mise en oeuvre du procédé selon l'une quelconque des revendications 1 à 4, comprenant une salle blanche (3), dans laquelle les récipients (B) sont remplis et peuvent être pré-fermés dans un poste de pré-fermeture (7) avec une première partie d'un système de fermeture, à savoir un opercule d'étanchéité, et comprenant un premier sas de nettoyage (13) pour les récipients (B) qui est associé à la salle blanche (3) de telle sorte que les récipients (B) sont nettoyés avant d'entrer dans la salle blanche (3), et un poste de fermeture à l'extérieur de la salle blanche (3), dans lequel les récipients (B) peuvent être définitivement fermés avec une deuxième partie du système de fermeture.

6. Dispositif selon la revendication 5, **caractérisé en ce qu'**il est prévu un deuxième sas de nettoyage (15) pour les systèmes de fermeture (V), lequel est associé à la salle blanche (3) de telle sorte que les systèmes de fermeture (V) sont nettoyés avant d'entrer dans la salle blanche (3).

7. Dispositif selon la revendication 5 ou 6, **caractérisé en ce que** le premier et/ou le deuxième sas de nettoyage (13;15) sont munis d'un dispositif de projection et/ou d'un dispositif d'injection de gaz.

8. Dispositif selon l'une quelconque des revendications 5 à 7, **caractérisé en ce que** le dispositif de projection projette sur les objets à nettoyer un liquide, un rayonnement UV, un rayonnement radioactif et/ou du gaz - en particulier de l'ozone.

9. Dispositif selon l'une quelconque des revendications 5 à 8, **caractérisé en ce qu'**il est prévu un poste de post-nettoyage (9; 11).
